# EUROPEAN PATENT SPECIFICATION

(11) **EP 1 900 326 B1**
(45) Date of publication and mention of the grant of the patent: **05.01.2011**
(21) Application number: 07017882.7
(22) Date of filing: 12.09.2007
(51) Int. Cl.: A61B 6/04, A61B 19/00

(54) **Support for holding and immobilizing a portion of the body of a living being for treatment**
Träger zum Halten und Fixieren eines Körperteils eines Lebewesens zu Behandlungszwecken
Support pour maintenir et immobiliser une portion d'un corps d'un être vivant en cours de traitement

(30) Priority: 12.09.2006 US 531099
(43) Date of publication of application: 19.03.2008
(73) Proprietor: Civco Medical Instruments Co., Inc., Kalona, IA 52247 (US)
(72) Inventor: de Mooy, Leo G., 2804 PD Gouda (NL); Schats, Vincent, 2562 JB 's Gravenhage (NL)
(74) Representative: Moore, Christopher Mark

(56) References cited:
- EP-A- 1 174 088
- WO-A-03/061477
- GB-A- 2 310 394
- US-A- 5 702 406
- US-A- 5 775 337
- US-A1- 2005 284 490

## Description

### BACKGROUND OF THE INVENTION

### 1. FIELD OF INVENTION

This invention relates to a positioning device for medical applications and more particularly to a device for supporting and immobilizing a portion, e.g., the head and neck, of the body of a person for treatment, e.g., radiotherapy treatment.

### 2. DESCRIPTION OF RELATED ART

The treatment of patients having cancer frequently makes use of radiation therapy wherein radiation is directed to a particular situs in the patient's body. One such type of radiation therapy is Intensity Modulated Radiation Therapy (IMRT). IMRT requires high precision and a reliable and accurate patient set-up to position and immobilize the relevant portion of the patient's body undergoing the radiation. Various devices and equipment are available for effecting such action. For example, patient couches or tables are commonly provided at the radiation machine, e.g., linear accelerator, CT machine, etc., to support the patient in a prone or supine position while the relevant portion of the patient's body is held in a fixed or immobilized condition. To that end the immobilization of the relevant portion of the patient's body is commonly achieved by various types of devices mounted on the patient couch/table.

For example, it is known to execute a head support by means of a device made up of plates pivotably supported by rods from the patient support table. The plates can be positioned against the head of a patient lying on the patient support table and they can be fixed in this position. In such a case, the patient's head will mostly rest on a somewhat resilient support, such as a cushion. Although the plates can be formed in such a way that they are somewhat adapted to the shape of a head, the device as such is not suitable for bringing the patient's head in exactly the same position during subsequent treatments. Thus, such head restraint devices are not suitable if a patient is to undergo treatments a considerable number of times, such as in case of radiation oncology, in which it is desirable that the head is in exactly the same position during each subsequent treatment.

Another head restraint device commonly utilized is a mask that is placed over the face of the patient to hold the patient's head stationary. Such masks may be molded to conform to the contours of the patient's face to ensure maximum immobilization. The back of the patient's head and contiguous portion of the patient's neck may be supported by a cushion which itself can be pre-contoured for a specific shape or can be conformed, e.g., molded, to the shape of the back of the patient's head. The mask itself can be pre-formed to a shape that will generally conform to the contours of the patient's face, or may be molded on the patient's face to closely conform to those contours. The molding of the mask is typically conducted preceding the first treatment. After this the mask can be mounted on the head of the patient and subsequently will be fixed to the patient support table. However, the patient's head and neck will still have to be supported, such as by cushions filled with granular material, for example, or by preformed cushions. Here, too, deviations in the position of the head in relation to the preceding treatment can easily occur.

BrainLAB, AG offers a system for immobilizing the head of a person. That system basically comprises use of two masks or sheets of thermoplastic material. In particular, a person who is to undergo radiation therapy is first disposed in a prone position and a first thermoplastic material sheet or mask is placed over the back of his/her head and neck while the his/her head and neck is supported by some other means to conform the sheet to the contours of the back portion of his/her head and neck. Once the first thermoplastic sheet has been conformed and has cured (set-up) it is inverted and the person is then disposed in a supine position on it so that his/her head neck is supported by it. A second thermoplastic sheet or mask is then used to conform to the contours of the person's face. After the face mask/sheet has cured the two sheets/masks can then be used as a unit to hold and immobilize the person's head and neck for radiation therapy or any other purpose. While the BrainLAB system is generally suitable for its intended purposes it leaves something to be desired from the standpoints of ease and simplicity of use and precise contouring since it requires positioning the person first in a prone position and then in a supine position to effect the formation of the two sheets/masks.

Sinmed BV, a company owned by CIVCO Medical Solutions, the assignee of this invention, offers equipment for patient positioning and immobilization which is highly effective and easy to use. For example, Sinmed BV offers a carbon fiber couch top under the trademark Mastercouch^{®} and head and neck fixation systems under the trademark Posifix®. The Posifix® head and neck positioning system is composed of several items, namely, different baseplates, varying from a basic acrylic head baseplate to more sophisticated carbon fiber IMRT baseplate that accommodates a wide variety of head, neck and shoulder masks. Each baseplate is indexed to the simulator, linear accelerator or CT couch by using aluminum fixation rails. A variety of simply attachable fixation brackets ensures a rigid lateral and longitudinal positioning of the baseplate onto any couch top. In addition, IMRT baseplates are mounted on and extend from the cranial end of the couch. A range of head supports, made of low density polyethylene foam, is available for comfortable and consistent prone and supine positioning. Each head support fits snugly into any of the Posifix® baseplates. The patient is immobilized by snapping a thermoplastic mask onto the baseplate with quick fasteners. This allows the technician to mold the mask perfectly around the facial contours of the patient.

US Patent 5,702,406 discloses a support for immobilizing a body portion of such kind.

Notwithstanding the foregoing a need still exists for a support system which is arranged to support a portion of a being's body, e.g., an individual's head and neck, to enable a mask to be molded over the supported portion, whereupon the portion of the being's body is effectively immobilized, without sacrificing comfort. The subject invention addresses that need. In addition overcomes many of the disadvantages of the prior art.

### BRIEF SUMMARY OF THE INVENTION

One aspect of this invention entails a device in the form of a support assembly for a portion, e.g., the head and neck, of the body of a living being to immobilize that body portion. The support is arranged to be mounted on a support surface, e.g., a patient table or couch, and comprises a concave shaped member formed of a conformable material, e.g., a somewhat resilient foamed plastic material, a holder for the concave shaped member and a cover sheet. The cover sheet is formed of a flexible, conformable material, e.g., polycaprolactone, and is arranged to be disposed over the concave shaped member, whereupon the body portion of the living being can be appropriately positioned and impressed onto the cover sheet and underlying concave shaped member to cause the cover sheet to conform to the shape of the body portion without danger of the cover sheet being overstretched or otherwise damaged (e.g., the person's head breaking through the cover sheet). The flexible, conformable material of the cover sheet is curable, whereupon when the cover sheet has cured it permanently retains the shape of the body portion impressed therein.

In accordance with one aspect of the device of this invention the cover sheet is formed of a thermoplastic material that is arranged to be heated to enable it to conform to the body portion and thereafter cool to cure it, whereupon it permanently retains the shape of the body portion impressed therein. In addition, the cover sheet includes fastening means located adjacent its periphery and which are arranged to secure the cover sheet to the holder, whereupon the heated cover sheet can be stretched and held over the concave shaped member to enable the portion of the being's body to be impressed therein to conform it to the shape of that body portion.

In accordance with still another aspect of the device this invention the support additionally includes a mask arranged to be releasably secured to it when the portion of the being's body is supported on the cover sheet, whereupon the portion of the being's body is immobilized therebetween. The mask is formed of a conformable, curable material that is arranged to be conformed to the shape of the portion of the being's body and thereafter cured to permanently retain that shape. The mask and the support member can then be used together to immobilize the portion of the living being's body disposed therebetween.

In accordance with another aspect of this invention there is provided a method for supporting a portion of the body of a living being to immobilize that body portion. The method entails providing a support comprising a concave shaped member formed of a conformable material and a cover sheet, said cover sheet being formed of a flexible, conformable material, disposing the cover sheet over the concave shaped member; impressing the portion of the body of the living being onto the cover sheet and underlying concave shaped member to cause the cover sheet to conform to the shape of the body portion, and curing the cover sheet to cause it to permanently retain that shape.

In accordance with another aspect of the method of this invention the cover sheet is a thermoplastic material which is heated to enable it to be conformed to the shape of the portion of the body of the living being and then allowed to cool to cure the cover sheet, whereupon it permanently retains the shape of the portion of the body of the living being.

In accordance with still another aspect of the method of this invention the portion of the body of the living being comprises the being's head and neck and the method additionally entails disposing the back of the being's head and neck on the cover sheet and placing a curable, conformable mask sheet over the face of the being, whereupon the conformable mask sheet can be conformed to the shape of the patient's face and cured to produce a mask permanently retaining the shape of the face of the being.

### BRIEF DESCRIPTION OF SEVERAL VIEWS OF THE DRAWINGS

The invention will be described in conjunction with the following drawings in which like reference numerals designate like elements and wherein:
Fig. 1 is an exploded isometric view of one exemplary embodiment of a support assembly constructed in accordance with the subject invention shown in position on a conventional patient support table or cushion;
Fig. 2 is an exploded isometric view similar to Fig. 1, but showing the support assembly with three of its components, i.e., the adapter plate, the holder and the concave cushion, in their assembled state mounted on the patient support table, and with one component, i.e., the cover sheet, being disposed over the assembled components;
Fig. 3 is an isometric view similar to Fig. 1, but showing the support assembly in its fully assembled position and ready to be used with a patient to conform the support to the shape of the back of the head and neck of a patient;
Fig. 4 is an exploded isometric view of plural sections of the concave cushion of the exemplary support assembly of Fig. 1;
Fig. 5 is an isometric view similar to Fig. 4, but showing the plural sections of the concave cushion juxtaposed immediately adjacent one another to complete the formation of the concave cushion;
Fig. 6 is an isometric view of the holder of the exemplary support assembly of Fig. 1;
Fig. 7 is an exploded isometric view of the holder and the adapter plate of the exemplary support assembly of Fig. 1;
Fig. 8 is an isometric view of the adapter plate of the exemplary support assembly of Fig. 1;
Fig. 9 is an isometric view of the cover sheet of the exemplary support assembly of Fig. 1;
Fig. 10 is an enlarged isometric view of a portion of the cover sheet of Fig. 1, namely, one of its profiles forming a handle/connector;
Fig. 11 is an isometric view of the holder and adapter plate of the exemplary embodiment of Fig. 1 shown assembled together, with one of the profiles of the cover sheet being shown in a position wherein it is ready to be connected to the adapter plate;
Fig. 12 is an isometric view of a portion of the profile shown in Fig. 11 after it has been secured to the adapter plate;
Fig. 13 is an exploded isometric view showing the adapter plate and the holder connected thereto ready to be mounted and secured to a base plate of a patient support table;
Fig. 14 is a vertical, transverse sectional view of the exemplary support assembly of Fig. 1 shown during the process of conforming its cover sheet to the contours of the back of the head and neck of a patient;
Fig. 15 is a view similar to Fig. 14 but showing a mask that has been disposed over the face of the patient whose head and neck is supported by the exemplary assembly of Fig. 1, with the mask being conformed to the contours of the patient's face so that the combination of the mask and the support assembly can be used to repeatedly immobilize the head and neck of the patient for treatment;

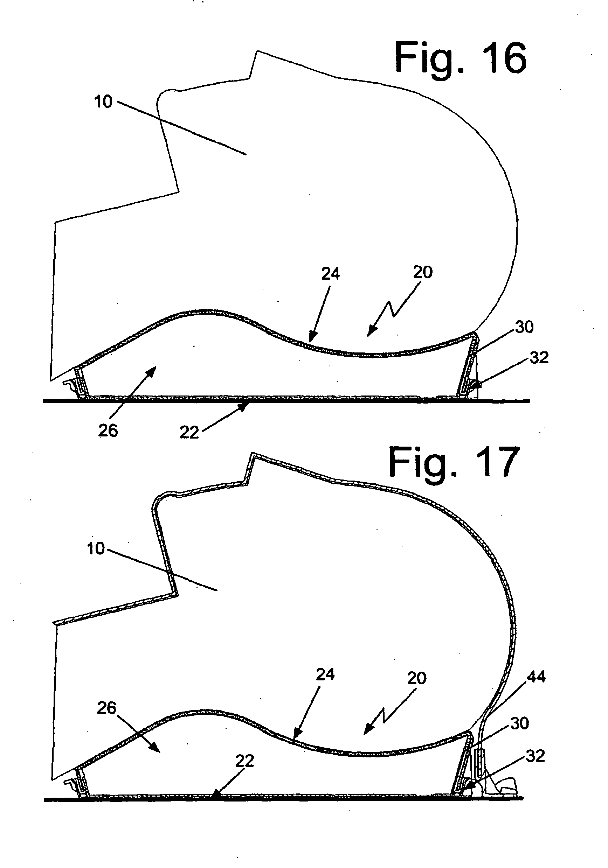

Fig. 16 is a vertical, longitudinal sectional view of the exemplary support assembly of Fig. 1 shown during the process of conforming its cover sheet to the contours of the back of the head and neck of a patient; and
Fig. 17 is a view similar to Fig. 16 but showing a mask that has been disposed over the face of the patient whose head and neck is supported by the exemplary assembly of Fig. 1, with the mask being conformed to the contours of the patient's face so that the combination of the mask and the support assembly can be used to repeatedly immobilize the head and neck of the patient for treatment.

### DETAILED DESCRIPTION OF THE INVENTION

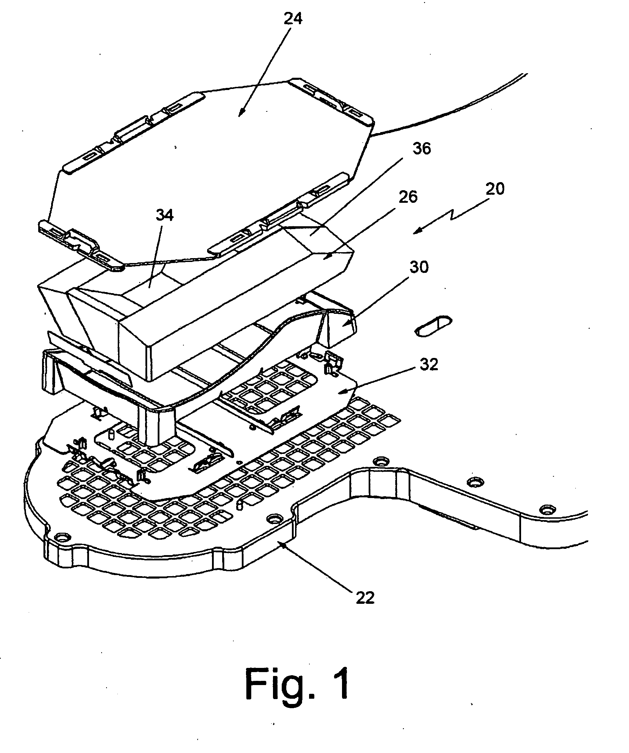

Referring now to the figures of the drawing, wherein like reference characters refer to like parts there is shown in Fig. 1 at 20 a support assembly for holding and immobilizing a portion of the body of a living being. The specific embodiment shown in Fig. 1 constitutes an occiput head and neck support, such as would be used to immobilize the head and neck of a person undergoing radiation therapy. It must be pointed out at this juncture that the support assembly 20 can be used to hold and immobilize any portion of the body of a living being for radiation treatment or any other type of therapy or for any other purpose for that matter, e.g., diagnostic scanning, etc. Thus, the occiput support assembly 20 shown herein is merely exemplary of various support assemblies that can be constructed in accordance with this invention. The details of the support assembly 20 will be described later. Suffice it for now to state that it is arranged to be releasably secured to any suitable support surface, such as base plate 22 for a patient support table or treatment couch (not shown) that is used with a radiation therapy machine or some other equipment, e.g., a CT scanner (not shown). The base plate 22 can be of any suitable conventional construction such as that sold by Sinmed BV under the trademark Posifix® of by other manufactures or suppliers (to be identified later).

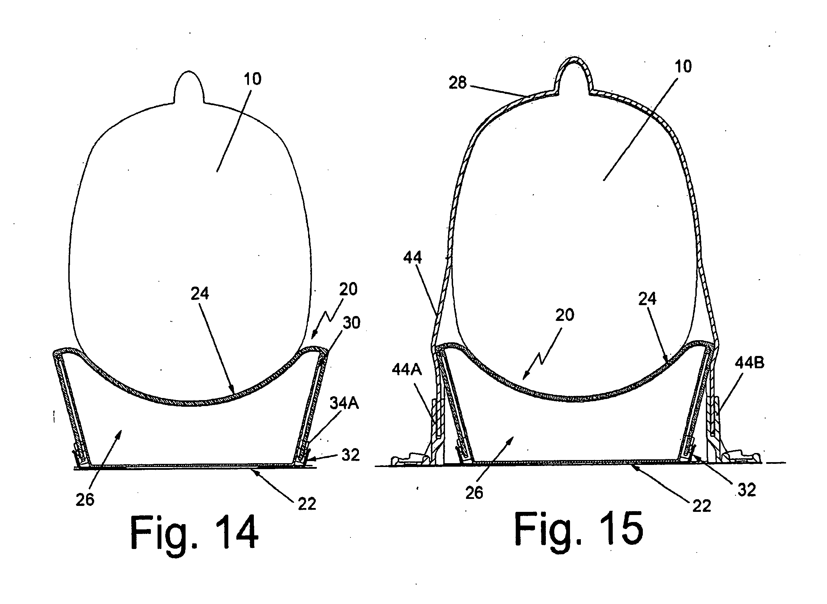

The support assembly 20 includes means, e.g., a cover sheet 24, that is adapted to be conformed to the contours of the relevant portion of the patient's body, e.g., the back of the head and neck. To accomplish that end the assembly 20 is mounted on the base plate 22 on a patient support table and the patient then lies in a supine position on the table so that the back of his/her head 10 and neck rests on the support assembly 20, and in particular its cover sheet 24 as shown in Fig. 14. The cover sheet 24 is itself supported by a conformable, resilient concave-shaped member or cushion 26. The cover sheet 24 is formed of a thermoplastic material and is arranged to be heated to enable it conform to the back portion of the patient's head and neck when the patient's head and neck are impressed downward onto the cover sheet. The concave-shaped cushion 26 is located below the cover sheet 24 and serves to provide some resistance to the impression of the patient's head and neck into the cover sheet, whereupon the cover sheet closely conforms to the contours of the back of the patient's head and neck. The cover sheet is then allowed to cool, whereupon it cures or sets to permanently retain its conformed shape. Once that has been accomplished a conventional mask 28, like that available commercially from Sinmed BV, can be formed on the patient's face to conform to the contours of the face while the patient's head 10 and neck is held on the support assembly 20 as shown in Fig. 15. The mask 28 will also be described later. Suffice to state that it is formed of a thermoplastic material that is arranged to be heated to soften it so that it can conform to the contours of the patient's face and then allowed to cool to cure and thereby permanently retain that shape. The conformed support 20 and the conformed mask 28 are arranged to be assembled and disassembled repeatedly so that they can be used together as a unit on the patient support table to hold the patient's head and neck in an immobilized state for radiation therapy or for any other purpose.

Referring now to Fig. 1 the more of the details of the construction and arrangement of the support assembly 20 will now be described. To that end the assembly 20 basically comprises the heretofore identified conformable cover sheet 24, the heretofore identified underlying resilient concave-shaped cushion 26, a holder 30, and an adapter plate 32. The holder 30 will be described in detail later. Suffice it for now to state that it is a tray-like member which serves a receptacle for the concave-shaped cushion 26. The housing 30 is adapted to be releasably secured to any conventional base plate 22, such as those offered commercially by MEDTEC, Inc. or other manufacturers or suppliers of base plates for use with patient tables or couches. Thus, the support assembly of this invention has wide applicability in that it can be used with any manufacturer's base plate/patent table. In order to enable such action various shaped adapter plates 32 are contemplated by this invention, with each adapter plate being particularly configured to enable it to be releasably secured to the particular base plate used.

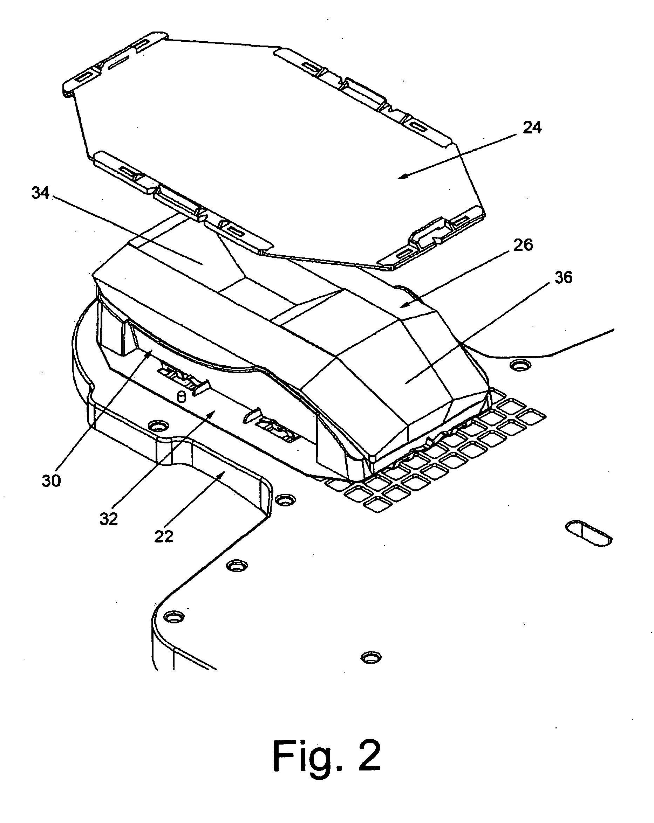

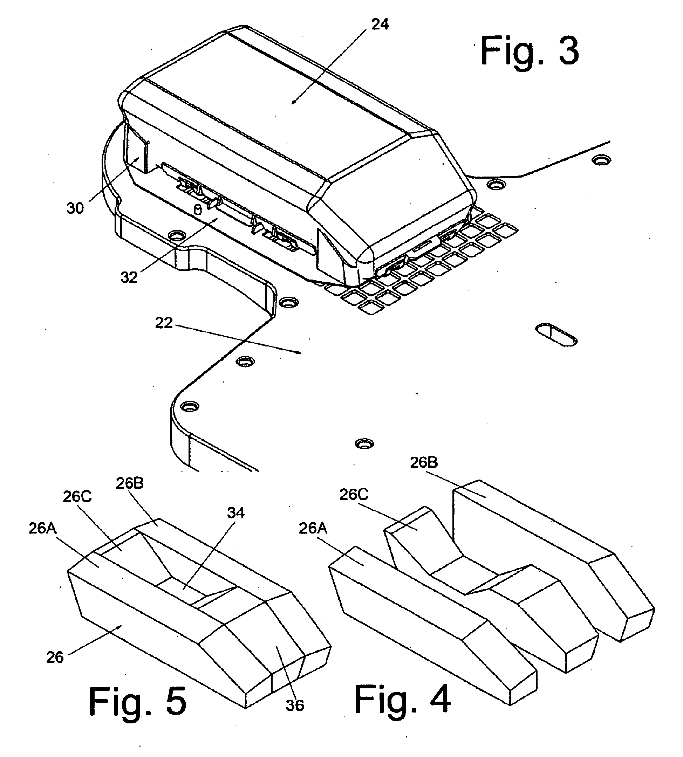

As best seen in Figs. 4 and 5 the resilient, concave-shaped cushion 26 is preferably composed of a plurality of sections 26A, 26B and 26C in the interest of economy of manufacture. Alternatively the cushion 26 may be made as a single integral unit or may be made of any number of sections. If made in sections the sections when juxtaposed as shown in Fig. 5 produce a unit having a recess or cavity 34 in its top surface and a downward sloping wall 36 extending from the recess to the front of the cushion. The cavity 34 is arranged to receive a portion of the back of the patient's head, while the sloping wall 36 receives the patient's neck when the patient is in a supine position on the patient table or couch. In accordance with the preferred embodiment the sections forming the concave member are formed of a conformable, resilient material, e.g., a phenolic foam with a closed cell structure. Suitable foam materials for the cushion 26 are available from Aspac Floral Foam Co., Ltd. under the trademark Bio-Foam. The sections 26A, 26B and 26C are arranged to be located within the hollow interior of the holder 30 as best seen in Fig. 2.

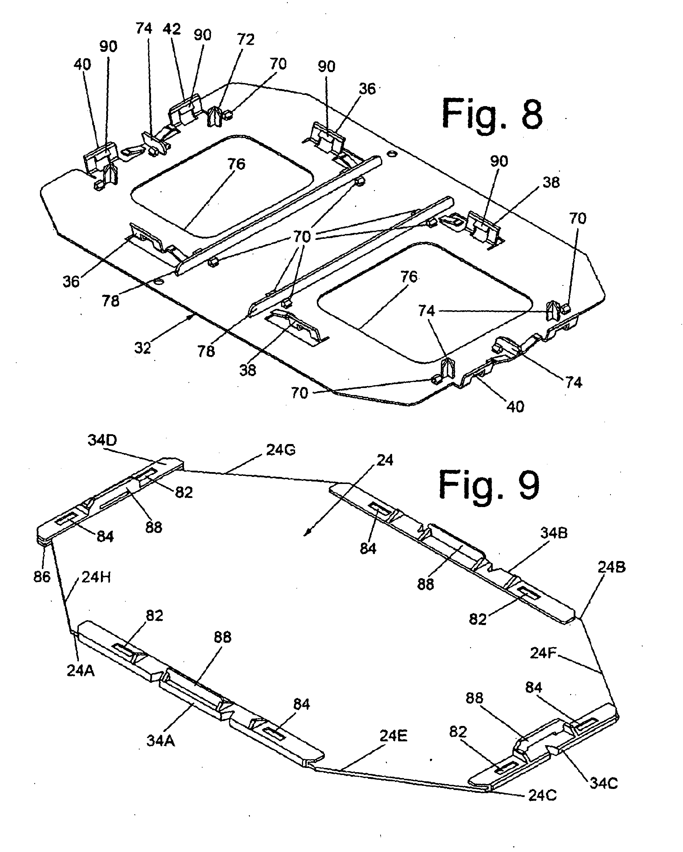

The cover sheet 24 is best seen in Fig. 9 and basically comprises a generally planar member formed of a conformable, curable, material which may be of a mesh-like construction or a solid web. In the exemplary embodiment shown the conformable curable material is a thermoplastic, e.g., a polycaprolactone homopolymer with a concentration of over 99% e-caprolactone, suitably for injection molding supplied by Sinmed BV under the trademark Posicast® or Solvay Interox Ltd. under the name Interox capa 650. Other suitable materials are available from Fluke Biomedical under the trademark "Halcyon", from Larson Medical Products, Inc. under the trademark Klarity®, from Orfit Industries under the trademarks UON and Efficast and from WFR/Aquaplast Corporation under the trademark Aquaplast. In the exemplary embodiment shown the cover sheet 24 is of a generally rectangular shape, having each of its corners cut away, thereby leaving four sides 24A, 24B, 24C and 24D and four angled corners 24E, 24F, 24G and 24H. It should be pointed out at this juncture that the shape of the cover sheet need not be like that shown in Fig. 9. Thus, the cover sheet need not have its corners cut away. In fact the cover sheet need not be rectangular, but can be any desired shape

A plurality of profiles in the form of handles 34A, 34B, 34C and 34D, each formed of a relatively rigid and strong plastic material, e.g., ABS, PS, PA or any other suitable moldable plastic, are fixedly secured to respective sides 24A, 24B, 24C and 24D of the cover sheet 24. The profiles 34A and 34B are relatively long and extend along the respective long sides 24A and 24B of the cover sheet. The profiles 34C and 34D are relatively short and extend along the respective short sides or ends 24C and 24D of the cover sheet. The profiles 34A and 34B are arranged to be releasably secured, e.g., snap fit, to respective connectors 36 and 38 (to be described later) forming a portion of the adapter plate 32. In a similar manner the profiles 34C and 34D are arranged to be releasably secured, e.g., snap fit, to respective connectors 40 and 42 (to be described later) forming a portion of the adapter plate 32.

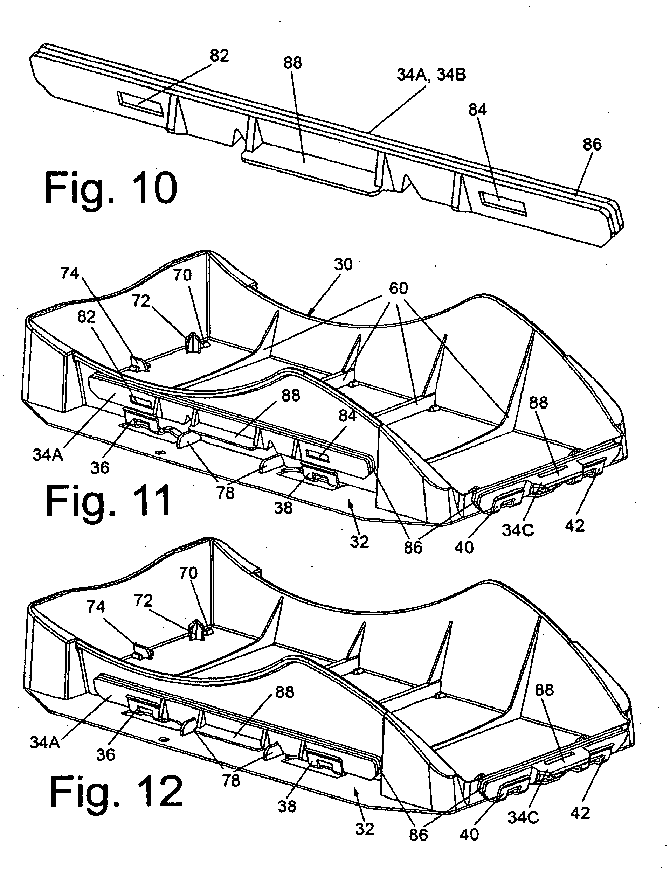

As mentioned above, the cover sheet 24 is arranged to be heated, e.g., immersed in hot water, to cause it to become soft and compliant so that it can be conformed to the portion of the patient's body that is impressed onto it. Thus, once the cover sheet has been appropriately heated, it can be juxtaposed over the support assembly 20 like shown in Fig. 2 and stretched over the top surface of the resilient concave-shaped cushion 26 by pulling on its profiles 34A - 34D and orienting them so that the profiles engage the connectors 36 - 42 of the adapter plate 32 as shown in Fig. 12. The support assembly is now ready to have the patient's head and neck contours impressed therein. To that end, the patient is placed on the patient support table so that the back of his/her head and neck rests on the heated and stretched cover sheet and the patient's head and neck is positioned as desired. Since the front portion 36 of the concave shaped cushion tapers downward it comfortably accommodates the neck of the patient. As downward pressure is applied onto the patient's head and neck it causes the surfaces of the back of the patient's head and neck to engage and depress the heated thermoplastic cover sheet 24. This causes the cover sheet to conform to the shape and position of the back of the patient's head and neck. At the same time the resilient foam material making up the cushion 26 applies a counter force to the downward pressure of the patient's head and neck to ensure that the cover sheet doesn't over-stretch or break while also ensuring that the cover sheet 24 closely conforms to the contours of the patient's head and neck. The cover sheet 24 is now allowed to cool. Once this has occurred the cover sheet "sets up" or cures, whereupon it permanently retains the shape corresponding to the contours of the patient's head and neck. The underlying cushion 26, which, as noted above is formed of foam, is retained in its conformed shape by the now cured, i.e., rigid, cover sheet. The support assembly 20 is now ready to enable a mask 28 to be formed for the patient's face. If desired, a coating of polyurethane (not shown) can be applied on the cover sheet to prevent adhesion of the patient's skin and/or hair to the cover sheet.

The mask 28 is preferably one that is sold by Sinmed BV as part of its Posifix® head and neck fixation systems or can be of any conventional construction, such as those sold by Fluke Biomedical, Larson Medical Products, Inc., Orfit Industries and WFR/Aquaplast Corporation. Thus, as shown in Fig. 15, the mask basically comprises a sheet 44 of a thermoplastic material. The sheet 44 may be of a net like construction or a web having a plurality of openings (not shown there) to enable the patient to breathe therethrough. The sheet 44 includes at least one relatively long profile in the form of a handle/connector. In the exemplary embodiment shown in Fig. 15 the mask 28 includes two such profiles, 44A and 44B, extending along and fixedly secured to the respective long side edges of the sheet 44. The profiles 44A and 44B are arranged to be releasably secured to the base plate 22 on the patient support table by respective conventional connectors (not shown). The mask 28 is conformed to the patient's face by heating it, e.g., immersing it in hot water, then placing it over the patient's face while the patient's head and neck are supported on the support assembly 20 and connecting the profiles 46 to the base plate 22. Since the support assembly has been configured to closely conform to the back of the patient's head and neck and to permanently retain that contour, considerable downward pressure can be applied to the conformable mask material to cause it to closely conform to the contours of the patient's face, i.e., the conformed support assembly will resist any downward movement of the patient's head and neck during the conforming of the mask to the patient's face. The mask is then allowed to cool, whereupon the contours formed into the mask set and the mask takes on a permanent fixed shape closely conforming to the face of the patient. The now rigid mask 28 can then be disconnected from the base plate and thereafter repeated used with the support assembly 20 whenever it is desired to immobilize that particular patient's head and neck for treatment.

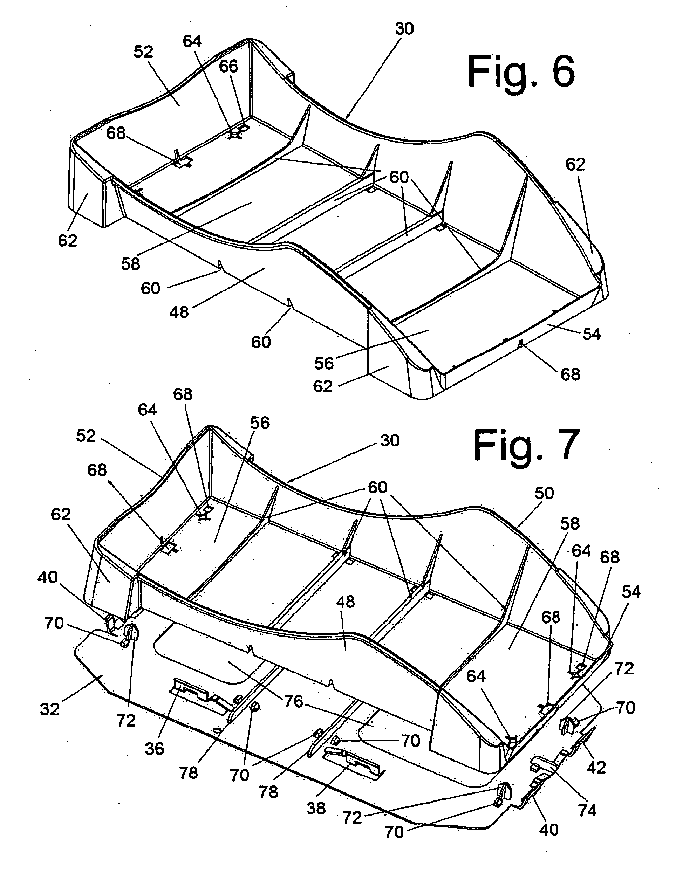

Referring now to Figs. 6, 7 and 8, the details of the holder 30 and the details of the adapter plate 32 will now be described. Thus, as can be seen the holder 30 basically comprises a hollow member of a tray-like construction made of any sufficient strong material, e.g., ABS, PS, PA or any other suitable moldable plastic, having a pair of long sidewalls 48 and 50, a rear wall 52 , a front wall 54 and a bottom wall 56, which together define a hollow interior cavity 58 for receipt of the sections 26A - 26D of the cushion 26. In order to reinforce the walls of the housing 30 and deter their deformation when pressure is applied to the support assembly 20 by the impression of the patient's head and neck into the softened cover sheet 24 and underlying cushion 26, a plurality of hollow reinforcing ribs 60 are molded into the bottom wall and sidewalls so that they extend transversely across the housing 30. Each corner of the housing is in the form of a downwardly extending leg 62. The bottom wall 56 of the housing includes plural openings 64, 66 and 68. The openings 66 are of square cross-sectional shape to receive complementary shaped connectors 70 (to be described later) of the adapter plate 32. The openings 64 are of a cruciform cross-sectional shape to receive complementary shaped cruciform connectors 72 (also to be described later) of the adapter plate 32. Lastly, the opening 68 are of a square shape with a longitudinally extending slot bisecting the square opening. The opening 68 is adapted to receive complementary shaped connector elements 74 (also to be described later) of the adapter plate 32.

As best seen in Figs. 7 and 8 the adapter plate 32 is a generally thin, planar member, formed of any suitable strong plastic material, e.g., ABS, PS or PA, and is of a generally rectangular shape having slightly cut-away corners and two generally square openings 76. A plurality of prong-like members project upward from the upper surface of the adapter plate. These prong-like members make up the heretofore identified cruciform connectors 72 and serve to help position the housing to the adapter plate. To that end, each of the cruciform shaped connectors 72 is arranged to be inserted in a respective cruciform shaped opening 64 when the housing is to be secured to the adapter plate. This action is accomplished by juxtaposing the housing 30 or the adapter plate 32 shown in Fig. 7 and then pressing down on the housing to cause the cruciform shaped connectors to enter their associated cruciform shaped openings in the bottom wall of the housing. The free or upper end of each of the cruciform shaped connectors is in the form of downwardly slanted cam surfaces to facilitate the entry of each cruciform connector into it respective opening and thereby guide the connection between the housing and the adapter plate. At least two elongated ribs 78 extend transversely across the adapter plate 32 on the top surface thereof. The ribs 78 are arranged to be received within the hollow undersurface of the reinforcing ribs 60 of the housing 30. In the exemplary embodiment the adapter plate includes two ribs 78 which are received in the two center-most reinforcing ribs 60. The connectors 70 are of a generally square cross sectional shape having a downwardly slanted cam surface extending from the free or upper end thereof and an undercut portion located under the cam surface. The connectors 70 are arranged to be extended through the square openings 66 in the bottom wall of the housing and to snap-fit therein. Thus, when the housing 30 in pressed downward with respect to the adapter plate to cause the cruciform shaped connectors to enter the cruciform shaped openings, the connectors 70 enter into the associated square shape openings in the bottom wall of the housing, with the downwardly sloping cam surface expediting such entry. This causes the free end portion of the connector to flex slightly at the undercut portion and then snap back into place to lock the connector 70 in its associated opening 66. The connector 74 basically comprises three components, namely, a short longitudinally extending linear finger projecting upward from the top surface of the adapter plate 32 and a pair of prong elements projecting upward from the top surface of the adapter plate on either side of the finger component. The prong components are shaped like the connectors 70. Thus, when the housing is secured to the adapter plate the finger component of the connector 74 enters the longitudinal slot portion of the opening 68 while each of the prong components of that connector enter the square portion of the opening 68 to snap-fit therein. The action of connectors 70, 72 and 74 entering the opening 66, 64 and 68, respectively, fixedly secures the holder 30 to the adapter plate 32.

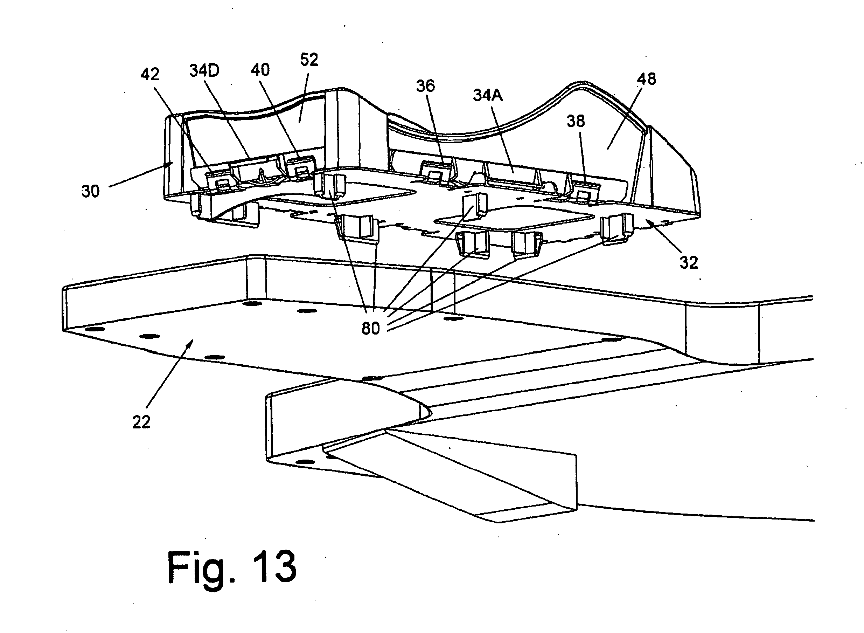

As mentioned earlier the adapter plate 32 is itself arranged to be releasably secured to the base plate 22 of the patient support table. This is accomplished by means of a plurality of prongs 80 which project downward from the undersurface of the adapter plate 32 as shown in Fig. 13. The prongs 80 are shaped and spaced from each other in accordance with the location of openings in the particular manufacturer's base plate to enable the adapter plate 32 of this invention to mount the support assembly of this invention on any type of base plate. For example, the adapter plate 32 shown in Figs. 7 and 8 is "MEDTEC, Inc." version of the adapter plate arranged to be used with base plates of MEDTEC, Inc., while the adapter plate shown in Fig. 13 is a "Sinmed, BV" version of the adapter plate arranged to be used with a Posifix® baseplate of Sinmed, BV. An "Orfit Industries" version of the adapter plate is configured to be used with an Orfit Industries base plate. The various versions of the adapter plate 32 all have the same upper features, but differ in the features, e.g., prongs 80, on the bottom of the adapter plate. Thus, since the details of the structure and arrangement of the various types of prongs 80 will ot be discussed further in the interests of brevity.

As mentioned above the profiles 34A - 34D forming the handles of the cover sheet 24 are arranged to be secured to the adapter plate 32 to secure the cover sheet over the concave-shaped cushion 26, whereupon the cover sheet can be conformed to the contours of the portion of the patient's body impressed therein. To that end, as best seen in Fig. 10 each profile 34A - 34D includes a pair of openings 82 and 84 to receive a respective connector elements forming a portion of the adapter plate 32. In particular, as will be described in more detail later, the openings 82 and 84 of the long profiles 34A and 34B are arranged to receive the connectors 36 and 38, respectively, of the adapter plate 32, while the openings 82 and 84 of the short profiles 34C and 34D are arranged to receive the connectors 40 and 42, respectively, of the adapter plate 32.

Turning now to Fig. 10 the details of the long profiles 34A and 34B will be discussed. Each profile 34A and 34B is an elongated member having a longitudinal slot 86 extending along its length. The free edge of a respective one of the long sides of the cover sheet 24 is fixedly secured in the slot 86. A flanged wall 88 extends perpendicularly to the portion of the profile forming the slot at a location immediately adjacent the bottom of the slot. The wall 88 serves as the handle portion of the profile. The heretofore identified openings 82 and 84 are each of rectangular shape and are located on opposite sides of the flanged wall and are each sized and shaped to receive a respective tab-like portions 90 (Fig. 8) of the connectors 36/38 of the adapter plate. The tab-like portions of the connectors 36/38 project at a generally downward acute angle and in an inward direction, i.e, toward the center of the adapter plate.

When either of the long sides of the cover sheet is to be connected to the adapter plate, the associated profile 34A or 34B is grasped by its handle portion 88 to move the profile to a position wherein its openings 82 and 84 are juxtaposed over a pair of upstanding connectors 36/38 of the adapter plate. The profile 34A is shown in this position in Fig. 11, with the cover sheet being omitted from this drawing in the interest of drawing simplicity. Downward movement of the long profile 34A or 34B causes it to engage the connectors 36/38, whereupon those connectors flex slightly outward so that their respective tab-like portions 90 snap-fit into respective ones of the openings 82 and 84, thereby locking the profile and its associated portion of the cover sheet to the adapter plate.

The profiles 34C and 34D are of a similar construction to profiles 34A and 34B, except that they are shorter in length and flanged wall 88 projects outward from the edge of the top or entrance to the slot 86 as best seen in Figs. 11 and 12. Thus, when either of the short sides of the cover sheet is to be connected to the adapter plate, the associated profile 34C or 34D is grasped by its/handle portion 88 to move the profile to a position wherein its openings 82 and 84 are juxtaposed over a pair of upstanding connectors 36/38of the adapter plate. Downward movement of the short profile 34A or 34B causes it to engage the connectors 40/42, whereupon those connectors flex slightly outward so that their respective tab-like portions 90 snap-fit into respective ones of the openings 82 and 84, thereby locking the profile and its associated portion of the cover sheet (not shown) to the adapter plate as shown in Figs. 11 and 12.

As should be appreciated from the foregoing the subject invention provides a support assembly, e.g., a head and neck support, which overcomes many of the drawbacks of the prior art, e.g., the patient's head will always take substantially the same position in each subsequent treatment, so that an optimum result of subsequent treatments can be achieved. Moreover, the subject invention enables the formation of the mask from a supine position, whereupon the conformed sheet and the underlying foam effectively support the head and neck of the patient. This should be contrasted with some prior art systems, such as sold by BrainLAB, AG as described above which requires molding the support for the back of the patient's head and neck from a prone position and then molding the mask for the patient's face from a supine position. Further still, by virtue of the intimate conformance of the support to the portion of the patient's body being supported and the large area of support, comfort is not sacrificed, e.g., the patient will not experience the support as being uncomfortable. Further still the assembly is relatively simple in construction, easy to use and is adaptable for use with various manufacturer's base plates and masks.

While the invention has been described in detail and with reference to specific examples thereof, it will be apparent to one skilled in the art that various changes and modifications can be made therein without departing from the scope thereof.

## Claims

1. A support (20) for a portion of the body of a living being to immobilize that body portion, said support (20) being arranged to be mounted on a support surface (22) and comprising a concave shaped member (26) and a cover sheet (24), said cover sheet (24) being formed of a flexible, conformable material and being arranged to be disposed over said concave shaped member (26), whereupon the portion of the body of the living being can be impressed and positioned onto said cover sheet (24) and underlying concave shaped member (26) to cause said cover sheet (24) to conform to the shape of that body portion while said concave shaped member (26) supports said cover sheet (24), said flexible, conformable material of said cover sheet (24) being curable, whereupon when said cover sheet (24) has cured it permanently retains the shape of that body portion, **characterised in that** the support (20) further comprises a holder (30) for said concave shaped member (26) wherein said holder (30) comprises a tray-like member having a pair of sidewalls (48, 50), a bottom wall (56), a rear wall (52) and a front wall (54) which together define a hollow interior (58) in which said concave shaped member (26) is located, said concave shaped member (26) being formed of conformable material and said holder (30) being arranged to confine said concave shaped member (26) to deter outward deformation thereof.

2. The support (20) of Claim 1 wherein said concave shaped member (26) is formed of a foamed plastic material.

3. The support (20) of Claim 2 wherein said foamed material comprises an expanded phenolic plastic.

4. The support (20) of Claim 1 wherein said cover sheet (24) is formed of a thermoplastic material arranged to be heated to enable it to conform to portion of the body of the living being and thereafter cool to cure it, whereupon it permanently retains that shape.

5. The support (20) of Claim 2 wherein said cover sheet (24) is formed of a thermoplastic material arranged to be heated to enable it to conform to the portion of the body of the living being and thereafter cool to cure it, whereupon it permanently retains that shape.

6. The support (20) of Claim 1 wherein said cover sheet (24) has a periphery (24A-H) and additionally comprises fastening means located adjacent said periphery, said fastening means (34A-D) being arranged to be secured to said holder (30) whereupon said cover sheet (24) can be stretched and held over said concave shaped member (26).

7. The support (20) of Claim 6 wherein said fastening means (34A-D) comprises plural bracket members, each of which is adapted to be secured to a respective portion of said holder 30.

8. The support (20) of Claim 1 wherein said concave shaped member 26 comprises an assembly of plural elements (26A-C).

9. The support (20) of Claim 1 wherein said concave shaped member (26) is located and held within said holder (30) and wherein said support (20) additionally comprises a mounting plate (32) for releasably securing said holder (30) to said support table (22).

10. The support (20) of Claim 1 additionally comprising a mask arranged to be releasably secured to said support when the portion of the being's body is supported on said cover sheet, whereupon the portion of the being's body is immobilized therebetween.

11. The support (20) of Claim 10 wherein said mask (28) is formed of a conformable, curable material that is arranged to be conformed to the shape of the portion of the being's body and thereafter cured to permanently retain that shape.

12. A method for supporting a portion of the body of a living being to immobilize that body portion, said method comprising:
A. providing a support (20) comprising a concave shaped member (26) and a cover sheet (24), said cover sheet (24) being formed of a flexible, conformable material;
B. disposing said cover sheet (24) over said concave shaped member (26);
C. impressing the body portion onto said cover sheet (24) and underlying concave shaped member (26) to cause said cover sheet (24) to conform to the shape and position of the body portion while said concave shaped member (26) supports said cover sheet (24);
D. curing said cover sheet (24) to cause said cover sheet (24) to permanently retain the shape conforming to the shape of the body portion of the living being;
and **characterised in that** the support (20) further comprises a holder (30) for said concave shaped member (26), wherein said holder (30) comprises a tray-like member having a pair of sidewalls (48, 50), a bottom wall (56), a year wall (52) and a front wall (54) which together define a hollow interior (58) in which said concave shaped member (26) is located, said concave shaped member (26) being formed of conformable material and said holder (30) being arranged to confine said concave shaped member (26) to deter outward deformation thereof.

13. The method of Claim 12 additionally comprising heating said cover sheet (24) to enable it to be conformed to the shape of the body portion of the living being.

14. The method of Claim 13 additionally comprising enabling said cover sheet (24) to cool to cure said cover sheet (24) whereupon said cover sheet (24) permanently retains the shape of the body portion.

15. The method of Claim 14 additionally comprising stretching said cover sheet (24) over said concave shaped member (26) to enable it to be conformed to the shape of the body portion, whereupon said cover sheet (24) is allowed to cool to cure and permanently retain the shape of the body portion.

16. The method of Claim 15 wherein the body portion comprises the being's head and neck.

17. The method of Claim 12 wherein the body portion comprises the being's head and neck and wherein said method additionally comprises:
E. disposing the back of the being's head and neck in the cover sheet (24); and
F. placing a conformable mask sheet (28) over the face of the being when the back of the being's head and neck is disposed on said support (20), whereupon said conformable mask sheet (28) can be conformed to the shape of the patient's face.

18. The method of Claim 17 wherein said conformable mask sheet (28) is curable and wherein said method additionally comprises curing said conformable mask sheet (28) to produce a mask permanently retaining the shape of the face of the being.

19. The method of Claim 18 additionally comprising securing said mask (28) to said support (20) with the being's head interposed therebetween to immobilize the being's head.

## Patentansprüche

1. Träger (20) für ein Körperteil eines Lebewesens, um dieses Körperteil zu immobilisieren, wobei der Träger (20) so angeordnet ist, dass er auf einer Trägerfläche (22) befestigbar ist und ein konkav geformtes Element (26) sowie ein Abdeckblatt (24) enthält, wobei das Abdeckblatt (24) als flexibles anpassbares Material ausgebildet ist und so angeordnet ist, dass es über das konkav geformte Element (26) legbar ist, woraufhin das Körperteil des Lebewesens auf das Abdeckblatt (24) gedrückt und dort positioniert wird und unter dem konkav geformten Element (26) liegt, so dass das Abdeckblatt (24) der Form des Körperteils folgt, während das konkav geformte Element (25) das Abdeckblatt (24) trägt, wobei das flexible anpassbare Material des Abdeckblatts (24) härtbar ist, woraufhin, wenn das Abdeckblatt (24) ausgehärtet ist, es die Form des Körperteils behält, **dadurch gekennzeichnet, dass** der Träger (20) ferner einen Halter (30) für das konkav geformte Element (26) enthält, wobei der Halter (30) ein wannenförmiges Element aufweist, das ein Paar von Seitenwänden (48, 50), eine Bodenwand (56), eine rückwärtige Wand (52) sowie eine vordere Wand (54) enthält, die zusammen ein hohles Inneres (58) definieren, in der das konkav geformte Element (26) angeordnet ist, wobei das konkav geformte Element (26) aus anpassbarem Material gebildet ist und der Halter (30) derart angeordnet ist, dass er das konkav geformte Element (26) einschließt, um dessen Deformation nach Außen zu verhindern.

2. Träger (20) nach Anspruch 1, bei dem das konkav geformte Element (26) aus geschäumtem Kunststoffmaterial gebildet ist.

3. Träger (20) nach Anspruch 2, bei dem das geschäumte Material expandiertes Phenoplast enthält.

4. Träger (20) nach Anspruch 1, bei dem das Abdeckblatt (24) aus einem thermoplastischen Material gebildet ist, das so angeordnet ist, dass es erwärmbar ist, um an das Körperteil des Lebewesens angepasst zu werden und danach zur Aushärtung zu kühlen ist, wonach es permanent diese Form behält.

5. Träger (20) nach Anspruch 2, bei dem das Abdeckblatt (24) aus einem thermoplastischen Material gebildet ist, das so angeordnet ist, dass es erwärmbar ist, um an das Körperteil des Lebewesens angepasst zu werden und danach zur Aushärtung abzukühlen, wonach es permanent diese Form behält.

6. Träger (20) nach Anspruch 1, bei dem das Abdeckblatt (24) einen Umfang (24A-H) aufweist und zusätzlich Befestigungsmittel enthält, die an der Peripherie angeordnet sind, wobei die Befestigungsmittel (34A-D) so angeordnet sind, dass sie an dem Halter befestigt sind, woraufhin das Abdeckblatt (24) gezogen und über dem konkav geformten Element (26) gehalten werden kann.

7. Träger (20) nach Anspruch 6, bei dem die Befestigungsmittel (34A-D) eine Mehrzahl von Halteklammern aufweisen, von denen jede an dem entsprechenden Teil des Halters (30) befestigt werden kann.

8. Träger (20) nach Anspruch 1, bei dem das konkav geformte Element (26) eine Zusammenstellung von mehreren Elementen (26A-C) enthält.

9. Träger (20) nach Anspruch 1, bei dem das konkav geformte Element (26) innerhalb des Halters (30) angeordnet und gehalten ist, und wobei der Träger (20) zusätzlich eine Befestigungsplatte (32) aufweist, um den Halter (30) an der Trägerplatte (22) lösbar zu halten.

10. Träger (20) nach Anspruch 1, der zusätzlich eine Maske aufweist, die so angeordnet ist, dass sie lösbar an dem Träger befestigt ist, wenn das Körperteil des Lebewesens auf der Abdeckung gehalten wird, wonach das Körperteil des Lebewesens dazwischen immobilisiert ist.

11. Träger (20) nach Anspruch 10, bei dem die Maske (28) aus anpassbarem, härtbarem Material gebildet ist, das so angeordnet ist, dass der Form des Körperteils des Lebewesens folgt und danach ausgehärtet wird, um die Form permanent zu erhalten.

12. Verfahren zur Stützung eines Körperteils eines Lebewesens, das zu immobilisieren ist, wobei das Verfahren Folgendes umfasst:
A. Bereitstellen eines Trägers (20), der ein konkav geformtes Element (26) sowie ein Abdeckblatt (24) enthält, wobei das Abdeckblatt (24) aus einem flexiblen anpassbarem Material besteht,
B. Ablage des Abdeckblatts (24) auf dem konkav geformten Element (26),
C. Aufdrücken des Körperteils auf das Abdeckblatt (24) und Unterlegen des konkav geformten Elements (26), so dass das Abdeckblatt (24) der Form und Position des Körperteils folgt, während das konkav geformte Element (26) das Abdeckblatt (24) trägt,
D. Aushärten des Abdeckblatts (24), so dass das Abdeckblatt (24) die Form behält, die der Form des Körperteils des Lebewesens folgt,
**dadurch gekennzeichnet, dass** der Träger (20) ferner einen Halter (30) für das konkav geformte Element (26) aufweist, wobei der Halter (30) ein wannenförmiges Element aufweist, das ein Paar von Seitenwänden (48, 50), eine Bodenwand (56), eine rückwärtige Wand (52) und eine Vorderwand (54) aufweist, die zusammen ein hohles Inneres (58) bilden, in dem das konkav geformte Element (26) angeordnet ist, wobei das konkav geformte Element (26) aus verformbarem Material gebildet ist und der Halter (30) so angeordnet ist, dass er das konkav geformten Element (26) einschließt, um eine Deformation nach außen zu verhindern.

13. Verfahren nach Anspruch 12, welches zusätzlich das Erwärmen des Abdeckblatts (24) umfasst, um es zu ermöglichen, dass es der Form des Körperteils des Lebewesens folgt.

14. Verfahren nach Anspruch 13, das zusätzlich die Möglichkeit umfasst, das Abdeckmaterial (24) zu kühlen, um das Abdeckblatt (24) auszuhärten, woraufhin das Abdeckblatt (24) permanent die Form des Körperteils behält.

15. Verfahren nach Anspruch 14, welches zusätzlich das Strecken des Abdeckblatts (24) über das konkav geformte Element (26) umfasst, um es zu ermöglichen, dass es der Form des Körperteils folgt, wonach das Abdeckblatt (24) abkühlen und aushärten kann und die Form des Körperteils permanent erhält.

16. Verfahren nach Anspruch 15, bei dem das Körperteil den Kopf und den Nacken eines Lebewesens umfasst.

17. Verfahren nach Anspruch 12, bei dem das Körperteil den Kopf und den Nacken des Lebewesens umfasst, wobei das Verfahren zusätzlich umfasst:
E. Anordnung der Rückseite des Kopfes und Nackens des Lebewesens in dem Abdeckblatt (24) und
F. Anordnung eines anpassbaren Maskenblatts (28) über dem Gesicht des Lebewesens, wenn die Rückseite des Kopfes und des Nackens des Lebewesens auf dem Träger (20) angeordnet ist, woraufhin das verformbare Maskenblatt (28) der Form des Gesichts des Patienten folgen kann.

18. Verfahren nach Anspruch 17, bei dem das verformbare Maskenblatt (28) aushärtbar ist, und wobei das Verfahren zusätzlich das Aushärten des verformbaren Maskenblatts (28) umfasst, um eine Maske zu erstellen, die die Form des Gesichts des Lebewesens permanent behält.

19. Verfahren nach Anspruch 18, das zusätzlich das Befestigen der Maske (28) am Träger (20) umfasst, wobei der Kopf des Lebewesens dazwischen angeordnet ist, um den Kopf des Lebewesens zu immobilisieren.

## Revendications

1. Support (20) pour une partie du corps d'un être vivant pour immobiliser cette partie de corps, ledit support (20) étant agencé pour être monté sur une surface de support (22) et comprenant un élément de forme concave (26) et une feuille de couverture (24), ladite feuille de couverture (24) étant constituée d'un matériau conformable souple et étant adaptée pour être disposée sur ledit élément de forme concave (26), après quoi la partie du corps de l'être vivant peut être imprimée et positionnée sur ladite feuille de couverture (24) et élément de forme concave sous-jacent (26) pour amener ladite feuille de couverture (24) à se conformer à la forme de cette partie de corps alors que ledit élément de forme concave (26) supporte ladite feuille de couverture (24), ledit matériau conformable souple de ladite feuille de couverture (24) étant durcissable, après quoi lorsque ladite feuille de couverture (24) a durci, elle conserve en permanence la forme de cette partie de corps, **caractérisé en ce que** le support (20) comprend en outre une monture (30) pour ledit élément de forme concave (26), dans lequel ladite monture (30) comprend un élément en forme de plateau ayant une paire de parois latérales (48, 50), une paroi inférieure (56), une paroi arrière (52) et une paroi avant (54) qui définissent ensemble un intérieur creux (58) dans lequel ledit élément de forme concave (26) est positionné, ledit élément de forme concave (26) étant constitué d'un matériau conformable, et ladite monture (30) étant agencé pour confiner ledit élément de forme concave (26) afin d'empêcher sa déformation vers l'extérieur.

2. Support (20) selon la revendication 1, dans lequel ledit élément de forme concave (26) est constitué d'une matière plastique alvéolaire.

3. Support (20) selon la revendication 2, dans lequel ladite matière alvéolaire comprend un plastique phénolique expansé.

4. Support (20) selon la revendication 1, dans lequel ladite feuille de couverture (24) est constituée d'un matériau thermoplastique adapté pour être chauffé afin de lui permettre de se conformer à la partie du corps de l'être vivant et ensuite refroidi pour le faire durcir, après quoi il conserve cette forme de manière permanente.

5. Support (20) selon la revendication 2, dans lequel ladite feuille de couverture (24) est constituée d'un matériau thermoplastique agencé pour être chauffé pour lui permettre de se conformer à la partie du corps de l'être vivant et ensuite refroidi pour le faire durcir, après quoi il conserve cette forme de manière permanente.

6. Support (20) selon la revendication 1, dans lequel ladite feuille de couverture (24) a une périphérie (24A-H) et comprend en plus des moyens de fixation positionnés de manière adjacente à ladite périphérie, lesdits moyens de fixation (34A-D) étant adaptés pour être fixés audit dispositif de maintien (30), après quoi ladite feuille de couverture (24) peut être étirée et maintenue sur ledit élément de forme concave (26).

7. Support (20) selon la revendication 6, dans lequel lesdits moyens de fixation (34A-D) comprennent plusieurs éléments de console, dont chacun est adapté pour être fixé sur une partie respective dudit dispositif de maintien (30).

8. Support (20) selon la revendication 1, dans lequel ledit élément de forme concave (26) comprend un ensemble de plusieurs éléments (26A-C).

9. Support (20) selon la revendication 1, dans lequel ledit élément de forme concave (26) est positionné et maintenu à l'intérieur dudit dispositif de maintien (30) et dans lequel ledit support (20) comprend en plus une plaque de montage (30) pour fixer de manière amovible ladite monture (30) sur ladite table de support (22).

10. Support (20) selon la revendication 1, comprenant en outre un masque adapté pour être fixé de manière amovible sur ledit support lorsque la partie du corps de l'être vivant est supportée par ladite feuille de couverture, après quoi la partie du corps de l'être vivant est immobilisée entre eux.

11. Support (20) selon la revendication 10, dans lequel ledit masque (28) est constitué d'un matériau conformable durcissable qui est agencé pour être conformé à la forme de la partie du corps de l'être vivant et ensuite durci pour conserver cette forme de manière permanente.

12. Procédé pour supporter une partie du corps d'un être vivant afin d'immobiliser cette partie de corps, ledit procédé comportant de :
A. prévoir un support (20) comprenant un élément de forme concave (26) et une feuille de couverture (24), ladite feuille de couverture (24) étant constituée d'un matériau conformable souple ;
B. disposer ladite feuille de couverture (24) sur ledit élément de forme concave (26) ;
C. imprimer la partie de corps sur ladite feuille de couverture (24) et élément de forme concave sous-jacent (26) pour amener ladite feuille de couverture (24) à se conformer à la forme et à la position de la partie de corps alors que ledit élément de forme concave (26) supporte ladite feuille de couverture (24) ;
D. faire durcir ladite feuille de couverture (24) pour amener ladite feuille de couverture (24) à conserver de manière permanente la forme se conformant à la forme de la partie de corps de l'être vivant ;
et **caractérisé en ce que** le support (20) comprend en outre une monture (30) pour ledit élément de forme concave (26), dans lequel ladite monture (30) comprend un élément en forme de plateau ayant une paire de parois latérales (48, 50), une paroi inférieure (56), une paroi arrière (52) et une paroi avant (54) qui définissent ensemble un intérieur creux (58) dans lequel ledit élément de forme concave (26) est positionné, ledit élément de forme concave (26) étant constitué d'un matériau conformable et ladite monture (30) étant agencé pour confiner ledit élément de forme concave (26) afin d'empêcher sa déformation vers l'extérieur.

13. Procédé selon la revendication 12, comprenant en outre l'étape consistant à chauffer ladite feuille de couverture (24) pour lui permettre de se conformer à la forme de la partie de corps de l'être vivant.

14. Procédé selon la revendication 13, comprenant en outre l'étape consistant à permettre à ladite feuille de couverture (24) de se refroidir, pour faire durcir ladite feuille de couverture (24), après quoi ladite feuille de couverture (24) conserve de manière permanente la forme de la partie de corps.

15. Procédé selon la revendication 14, comprenant en outre l'étape consistant à étirer ladite feuille de couverture (24) sur ledit élément de forme concave (26) pour lui permettre d'être conformée à la forme de la partie de corps, après quoi, ladite feuille de couverture (24) est laissée refroidir pour durcir et conserver de manière permanente la forme de la partie de corps.

16. Procédé selon la revendication 15, dans lequel la partie de corps comprend la tête et le cou de l'être vivant.

17. Procédé selon la revendication 12, dans lequel la partie de corps comprend la tête et le cou de l'être vivant et dans lequel ledit procédé comporte en outre de :
E. disposer l'arrière de la tête et du cou de l'être vivant dans la feuille de couverture (24), et
F. placer une feuille de masque conformable (28) sur le visage de l'être vivant lorsque l'arrière de la tête et du cou de l'être vivant est disposé sur ledit support (20), après quoi ladite feuille de masque conformable (28) peut se conformer à la forme du visage du patient.

18. Procédé selon la revendication 17, dans lequel ladite feuille de masque conformable (28) est durcissable et dans lequel ledit procédé comprend en outre l'étape consistant à faire durcir ladite feuille de masque conformable (28) pour produire un masque qui conserve de manière permanente la forme du visage de l'être vivant.

19. Procédé selon la revendication 18, comprenant en outre l'étape consistant à fixer ledit masque (28) sur ledit support (20) avec la tête de l'être vivant intercalée entre eux afin d'immobiliser la tête de l'être vivant.
